# EUROPÄISCHE PATENTSCHRIFT

(11) **EP 2 033 605 B1**
(45) Veröffentlichungstag und Bekanntmachung des Hinweises auf die Patenterteilung: **28.03.2012**
(21) Anmeldenummer: 07115424.9
(22) Anmeldetag: 31.08.2007
(51) Int. Cl.: A61F 9/01

(54) **Lasersystem zum Ablatieren von Hornhaut an einem Patientenauge**
Laser system for ablating the cornea of a patient's eye
Système laser pour l'ablation de cornée dans un oeil de patient

(43) Veröffentlichungstag der Anmeldung: 11.03.2009
(73) Patentinhaber: Schwind eye-tech-solutions GmbH & Co. KG, 63801 Kleinostheim (DE)
(72) Erfinder: Arba-Mosquera, Samuel, 63739 Aschaffenburg (DE)
(74) Vertreter: Hofstetter, Alfons J.

(56) Entgegenhaltungen:
- EP-A- 1 364 632
- EP-A- 1 591 087
- WO-A-94/15238
- US-A- 6 099 522

## Beschreibung

Die Erfindung betrifft ein Lasersystem zum Ablatieren von Hornhaut an einem Patienten auge sowie ein Verfahren zum Vorbereiten einer Ablation von Hornhaut, bei dem ein spezifisches derartiges Lasersystem eingesetzt wird.

Naturgemäß umfasst das Lasersystem eine Laserquelle. Beim Ablatieren der Hornhaut muss der Laserstrahl, den die Laserquelle erzeugt, auf unterschiedlichen Stellen der Hornhaut auftreffen. Als Zieleinrichtung fungiert eine Scannereinrichtung, die den Laserstrahl ablenkt, damit er eben auf jeweils einer ganz bestimmten Stelle auftrifft. Damit nun der behandelnde Arzt von vorne auf das Patientenauge sehen kann, darf die Scannereinrichtung nicht vor dem Auge angeordnet sein. Aus diesem Grund wird der von der Scannereinrichtung ausgelenkte Laserstrahl von einem Spiegel reflektiert. Dieser Spiegel ist üblicherweise halbdurchlässig, so dass der behandelnde Arzt durch den Spiegel hindurch auf das Auge sehen kann. Der Spiegel hat eine ebene Oberfläche. Die Scannereinrichtung lenkt den Laserstrahl von einem bestimmten Ursprungspunkt aus auf unterschiedliche Stellen dieser ebenen Spiegeloberfläche. Wird der Laserstrahl seitlich abgelenkt im Verhältnis zu einem nicht abgelenkten Zentralstrahl, so entfernt sich der seitliche Laserstrahl immer mehr von dem Zentralstrahl, je näher er dem Spiegel kommt. Wegen der ebenen Oberfläche des Spiegels setzt sich dies jenseits des Spiegels fort: Der seitlich abgelenkte Strahl entfernt sich noch weiter von dem Zentralstrahl, bis er auf dem Patientenauge auftrifft. Da die Oberfläche der Hornhaut konvex ist, bedingt dies zwingend, dass die seitlich abgelenkten Laserstrahlen nicht-senkrecht auf der Hornhautoberfläche auftreffen.

Nun ist die so genannte Ablationstiefe, d. h. die Tiefe, um die die Hornhaut durch einen einzelnen Laserpuls abgetragen wird, vom natürlichen Logarithmus der Intensität des Laserstrahls abhängig. Diese Intensität ist wiederum vom Auftreffwinkel des Laserstrahls auf der Hornhautoberfläche abhängig. Bei herkömmlichen Lasersystemen bedeutet dies, dass wegen des nicht-senkrechten Auftreffens der seitlich abgelenkten Laserstrahlen diese eine geringere Ablationstiefe haben als ein zentraler Laserstrahl. Es muss nun gewährleistet sein, dass ein vorbestimmtes Ablationsprofil präzise in die Hornhaut durch Abtragen derselben eingeschrieben wird. Würde man den Effekt des nicht-senkrechten Auftreffens der seitlich abgelenkten Laserstrahlen vernachlässigen, kann es zu Abweichungen von dem Wunschprofil kommen.

Man hat bisher erwogen, das Sollablationsprofil, von dem eine Steuereinheit des Lasersystems ausgeht, zu ändern, um den Effekt des nicht-senkrechten Auftreffens der seitlich abgelenkten Laserstrahlen vorab zu kompensieren. Dieses Sollablationsprofil ist dann gar nicht das echte Sollablationsprofil. Man kann alternativ auch bei gegebenem Ablationsprofil den Effekt des nicht-senkrechten Aufreffens der seitlich abgelenkten Laserstrahlen dadurch ausgleichen, dass das Geringerwerden der Intensität bei seitlicher Ablenkung durch eine Erhöhung der Zahl der Pulse, welche zur Seite hin gesandt werden, kompensiert wird. Die beiden genannten Lösungen haben den Nachteil, dass das gesamte Behandlungsverfahren, und insbesondere die Ablationsprozedur selbst, länger dauert. Es ist nicht wünschenswert, wenn die Ablationsprozedur zu lange dauert.

Man behilft sich zum Ausgleichen des genannten Effekts auch damit, die Hornhautoberfläche zu aplanieren. Das Auge wird hierbei gegen einen geeigneten lichtdurchlässigen Gegenstand gedrückt. Hierbei wird jedoch der Augeninnendruck erhöht, was störend für den Patienten ist und sogar schädlich sein kann.

Die WO 94/15238 beschreibt ein Verfahren zum Formen eines Laserstrahls. Der Laserstrahl wird hierbei in seiner Gesamtheit auf einen gekrümmten Spiegel geleitet, der den Laserstrahl auf einen Brennpunkt fokussiert. Einzelne Bereiche des Laserstrahls treffen auf unterschiedlichen Stellen des gekrümmten Spiegels auf. Die WO 94/15238 nennt als Anwendung die Bearbeitung von Werkstücken so wie auch von Gewebe eines Lebewesens, wie beispielsweise dessen Hornhaut.

Das US-Patent 6,099,522 beschreibt ein automatisch arbeitendes Lasersystem für chirurgische Zwecke. Bei einer Ausführungsform ist zwischen einem Spiegel, der einen Laserstrahl aus dem System auskoppelt, und einem Patientenauge eine Fokussierlinse angeordnet. Deren Stellung kann längs eines Pfades verschoben werden.

Die EP 1 591 087 A1 beschreibt eine Vorrichtung zur Laserbehandlung eines Auges, bei dem ein Laserstrahl von einer Laserquelle auf eine Zieleinrichtung gesandt wird, die den Laserstrahl auf unterschiedliche Stellen eines dichroitischen Spiegels lenkt, von wo aus er zu einer Linse reflektiert wird, die den Strahl auf ein Patientenauge fokussiert.

Die EP 0 326 760 A2 offenbart eine Vorrichtung zur Laserbehandlung eines Patientenauges, bei der im Strahlengang des Lasers zwei galvanometrischen Spiegeln als Scannereinrichtungen ein aktiver Spiegel zum Ausgleich von Aberrationsfehlern und zur Berücksichtigung individueller Eigenheiten der Patientenhomhaut nachgeordnet ist. Im Strahlengang folgt dem aktiven Spiegel eine aufwendige Optik, zu der ein verschwenkbarer Spiegel gehört. Durch dessen Verschwenken sollen die Laserstrahlen jeweils optimal auf die Hornhautoberfläche fokussiert werden. Die zweiteilige Form der unabhängigen Ansprüche basiert auf diesem Dokument.

Es ist Aufgabe der vorliegenden Erfindung, einen Weg aufzuzeigen, wie das oben geschilderte Problem so gelöst werden kann, dass die Behandlung möglichst effizient durchgeführt werden kann.

Die Aufgabe wird durch ein Lasersystem mit den Merkmalen gemäß Patentanspruch 1 in einem ersten Aspekt und mit den Merkmalen gemäß Patentanspruch 7 in einem zweiten Aspekt gelöst.

Gemäß dem ersten Aspekt der Erfindung und Alternative a) wird ein Spiegel gewählt, dessen Spiegeloberfläche nicht eben ist. Die nicht ebene Spiegeloberfläche lässt sich insbesondere passend zur typischen Form einer Hornhautoberfläche wählen, damit bei vorbestimmter Platzierung des Patienten in dem Lasersystem (wobei sich der Patient z. B. mit dem Kinn in einer Ablage abstützt und mit der Stirn an einem Anschlag nach vorne anlehnt) die seitlich abgelenkten Laserstrahlen senkrecht auf der Hornhautoberfläche auftreffen. Erfindungsgemäß ist zumindest das Erfordernis erfüllt, dass sie mit einem solchen Auftreffwinkel auftreffen, der näher an 90° ist, als es beim ebenen Spiegel der Fall ist.

Die Spiegeloberfläche kann beispielsweise sphärozylindrisch und insbesondere sphärisch gewählt sein. Unter bestimmten Umständen kann sich auch ein asphärischer Spiegel, insbesondere ein ellipsoidaler, als sinnvoll erweisen.

Gemäß Alternative b), bei der die individuellen Eigenschaften der Hornhautoberfläche eines Patientenauges besonders gut berücksichtigt werden können, ist vorgesehen, dass die Spiegeloberfläche veränderlich einstellbar ist, z. B. auf Anweisung durch dieselbe Steuereinheit, die auch die Scannereinheit ansteuert. Einen solchen Spiegel bezeichnet man auch als aktiven oder adaptiven Spiegel. Zur Spiegeloberfläche gehört hierbei eine Vielzahl von Stellelementen, z. B. Piezoaktoren, die auf Teiloberflächen des Spiegels einwirken und diese verstellen. Jedes Stellelement ist einzeln ansteuerbar, so dass die Spiegeloberfläche ganz genau so gestaltet werden kann, dass ein Optimum erreicht wird, was das senkrechte Auftreffen des Laserstrahls auf den unterschiedlichen Stellen der Hornhaut betrifft. Somit lässt sich das Verfahren zum Vorbereiten einer Ablation von Hornhaut an einem Auge eines Patienten nach Patentanspruch 9 durchführen, bei dem zunächst die Kontur der Hornhautoberfläche des Patientenauges vermessen wird und dann die Spiegeloberfläche derart eingestellt wird, dass ein aus dem Lasersystem austretender Laserstrahl (der den Scanner und die Spiegeloberfläche durchlaufen hat) bei vorbestimmter Platzierung des Patienten relativ zu dem Lasersystem (z. B. wie oben erwähnt Kinn und Stirn abgestützt), unabhängig von der Einstellung der Scannereinrichtung mit einem so nahe wie möglich an 90° liegenden Auftreffwinkel, bevorzugt genau senkrecht auf der Hornhautoberfläche auftreffen wird.

Bei einer bevorzugten Ausführungsform des erfindungsgemäßen Lasersystems ist dem Spiegel auch eine Linse nachgeordnet. Eine solche Linse kann zusätzlich Einfluss auf den Strahlengang nehmen.

Bei dem zweiten Aspekt der Erfindung wird ebenfalls bewirkt, dass im Vergleich zur Verwendung des ebenen Spiegels bei vorbestimmter Platzierung des Patienten der Auftreffwinkel des Laserstrahls näher an 90° liegt. Dies wird dadurch ermöglicht, dass dem Spiegel eine solche Linse nachgeordnet wird, die eine veränderliche Form hat.

Damit lässt sich das Verfahren nach Patentanspruch 10 durchführen, bei dem nach dem Vermessen der Kontur der Hornhautoberfläche des Auges der Abstand der Linse und/oder die Form der Linse derart eingestellt wird, dass wieder das Optimum erreicht wird, was den Auftreffwinkel des Laserstrahls angeht, und zwar für die Gesamtheit der Einstellungen der Scannereinrichtung betrachtet.

Selbstverständlich können die beiden Aspekte der Erfindung miteinander kombiniert werden, d. h. eine nicht ebene Spiegeloberfläche kann bei einem Lasersystem verwendet werden, das eine Linse aufweist, deren Abstand zum Spiegel und/oder deren Form veränderlich ist.

Nachfolgend werden bevorzugte Ausführungsformen unter Bezug auf die Zeichnung beschrieben, in der
- Fig.: 1 ein Lasersystem zum Ablatieren von Hornhaut an einem Patientenauge gemäß dem Stand der Technik schematisch veranschaulicht,
- Fig. 2: ein Lasersystem zum Ablatieren von Hornhaut an einem Patientenauge gemäß einem ersten Aspekt der Erfindung schematisch veranschaulicht,
- Fig. 3: ein Lasersystem zum Ablatieren von Hornhaut an einem Patientenauge gemäß einem zweiten Aspekt der Erfindung schematisch veranschaulicht,
- Fig. 4: ein Lasersystem zum Ablatieren von Hornhaut an einem Patientenauge gemäß einem dritten Aspekt der Erfindung schematisch veranschaulicht.

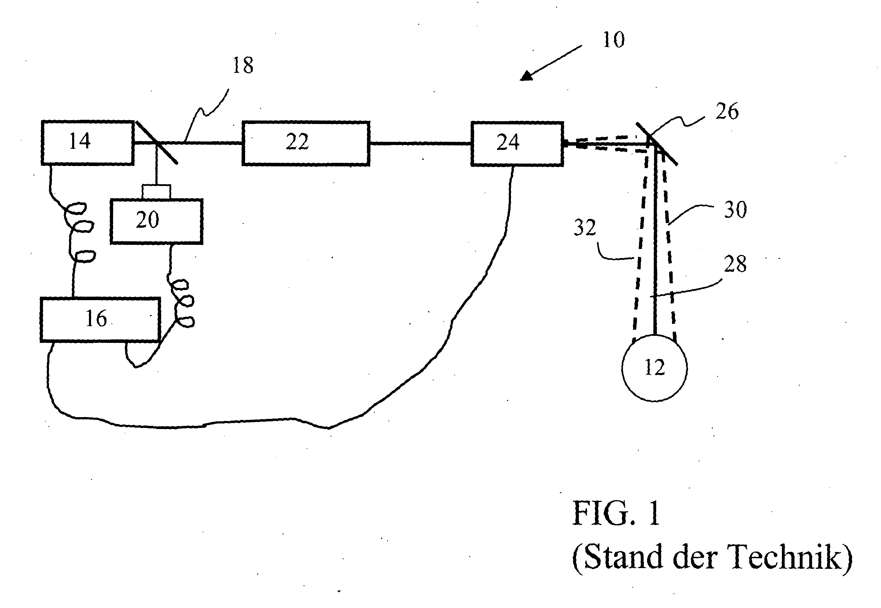

Ein bekanntes, in Fig. 1 gezeigtes und dort im Ganzen mit 10 bezeichnetes Lasersystem dient zum Ablatieren von Hornhaut an einem Auge 12 eines Patienten. Die Ablation erfolgt mit Hilfe eines Laserstrahls, der von einer Laserquelle 14 erzeugt wird. Die Laserquelle 14 wird von einer Steuereinheit 16 angesteuert. In dieser Steuereinheit ist ein so genanntes Ablationsprofil zu dem Patientenauge 12 abgelegt, das beschreibt, welche Sollkontur das Patientenauge hat, d. h. wie es nach der Behandlung aussehen soll. Zu diesem Ablationsprofil werden Pulse von Laserstrahlen 18 erzeugt, deren Energieinhalt mit Hilfe eines Energiesensors 20 überwacht wird, der mit der Steuereinheit 16 verbunden ist, damit eine mögliche Korrektur der Zahl der Pulse stattfinden kann. Die Laserstrahlen werden von so genannten Strahlformungsoptiken 22 in an sich bekannter Weise geformt und einer Scannereinrichtung 24 zugeleitet. Unter Scannereinrichtung ist jede Einrichtung zu verstehen, die den Laserstrahl 18 in unterschiedliche Richtungen wahlweise auslenken kann. Typischerweise umfasst die Scannereinrichtung 24 einen ersten Spiegel für die Auslenkung in einer ersten Richtung (x-Richtung) und einen zweiten Spiegel für die Auslenkung in einer zweiten Richtung (y-Richtung). Die Steuereinheit 16 ist mit der Scannereinrichtung 24 verbunden und steuert diese an. Entsprechend dem Ablationsprofil wird durch die Steuereinheit 16 festgelegt, wie oft der Laserstrahl an welcher Stelle auf der Hornhaut des Patientenauges 12 auftreffen soll. Jede Auslenkung der Scannereinrichtung 24 entspricht hierbei grundsätzlich einer Stelle auf dem Patientenauge 12: Der Laserstrahl wird von der Scannereinrichtung 24 nämlich über einen ebenen Spiegel 26 zum Patientenauge 12 gelenkt. Ein Zentralstrahl 28, z. B. der von der Scannereinrichtung 24 nicht abgelenkte ursprüngliche Strahl trifft senkrecht auf dem Patientenauge 12 auf. Seitlich abgelenkte Strahlen 30 und 32 entfernen sich vor und nach dem ebenen Spiegel 26 von dem Zentralstrahl 28. Sie treffen nicht-senkrecht auf dem Patientenauge 12 auf. Wie jedermann von der Intensität der Sonnenstrahlung her bekannt, hängt die Intensität von auftreffendem Licht von dem Auftreffwinkel ab. Somit ist die Intensität des Zentralstrahls 28 höher als die der seitlich abgelenkten Strahlen 30 und 32. Dadurch verringert sich die Ablationstiefe des Laserstrahls zur Seite hin, d. h. der Laser trägt umso weniger Hornhaut ab, je entfernter er vom Zentrum auftrifft.

Dieser Effekt soll durch die Erfindung ausgeglichen werden.

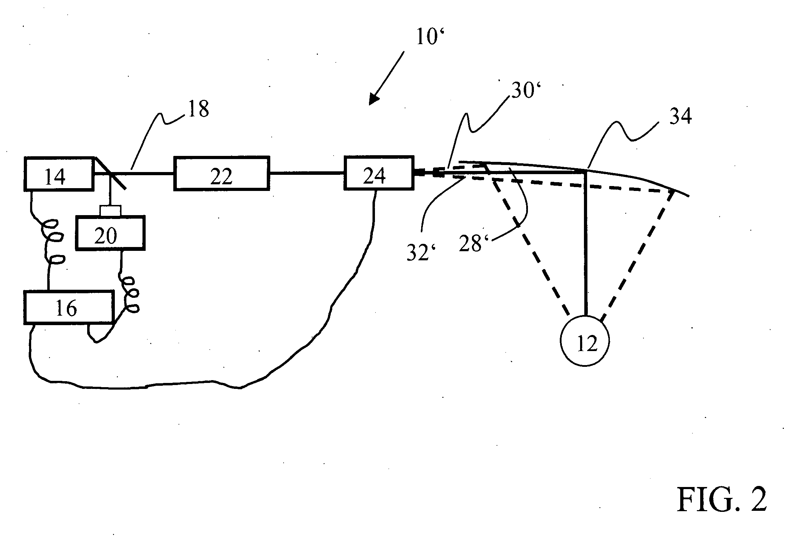

Fig. 2 zeigt ein Lasersystem 10' gemäß einem ersten Aspekt der Erfindung. Bauteile, die gegenüber dem Lasersystem 10 des Standes der Technik aus Fig. 1 unverändert sind, tragen dieselben Bezugszeichen. Bei dem Lasersystem 10' ist nun der Spiegel durch einen nicht ebenen Spiegel ersetzt. Die Nichtebenheit ist so gestaltet, dass nicht nur der Zentralstrahl 28' senkrecht auf der Hornhautoberfläche des Patientenauges 12 auftrifft, sondern auch die seitlich abgelenkten Strahlen 30' und 32' jeweils senkrecht auf der Hornhaut des Auges 12 auftreffen. Damit erzielt man für jeden Punkt auf der Hornhautoberfläche dieselbe Ablationstiefe, so dass das Ablationsprofil präziser eingeschrieben werden kann. Der Spiegel 34 mit der nicht ebenen Spiegeloberfläche kann ein sphärozylindrischer und insbesondere sphärischer Spiegel, ein asphärischer oder ein ellipsoidaler Spiegel sein. Gegebenfalls kann vorgesehen sein, dass der Spiegel 34 austauschbar ist, damit zu einem Patientenauge 12 ein passender Spiegel ausgewählt werden kann. Auch, wenn der Spiegel 34 verkippbar ist, ändert sich der Strahlengang, so dass auch durch ein Verkippen eine Anpassung an die Oberfläche der Hornhaut des Patientenauges vorgenommen werden kann.

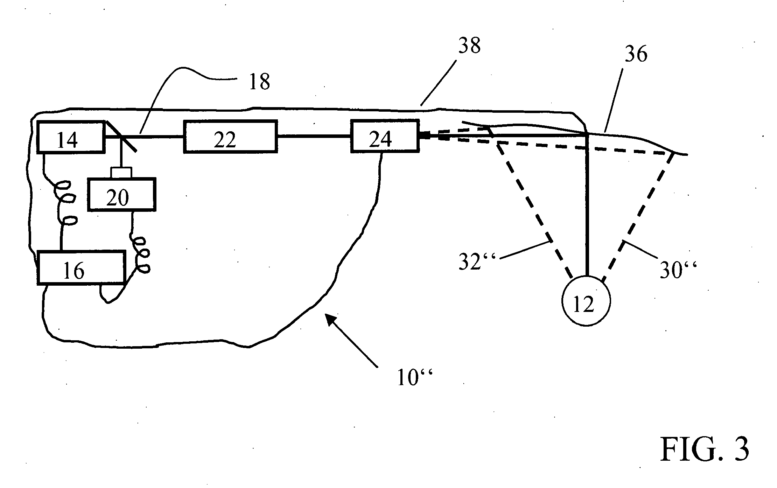

In Fig. 3 ist ein Lasersystem 10" gemäß einer zweiten Ausführungsform der Erfindung dargestellt. Dieses unterscheidet sich von der ersten Ausführungsform 10' dadurch, dass nunmehr ein nicht ebener Spiegel 36 verwendet wird, dessen Spiegeloberfläche veränderlich einstellbar ist. Solche aktiven oder adaptiven Spiegel sind bekannt. Typischerweise umfassen sie eine Mehrzahl von Stellelementen, z. B. Piezoelementen oder Kleinmotoren, die die Spiegeloberfläche lokal ändern können. Die Verstellelemente können über die Steuereinheit 16 angesteuert werden, indem sie über eine Verbindungsleitung 38 mit dieser verbunden werden.

Bei dieser Ausführungsform ist es möglich, die Spiegeloberfläche individuell passend zur Kontur der Hornhaut des Patientenauges 12 einzustellen. Diese muss vorher mit geeigneten Mitteln vermessen werden, und das Messergebnis muss der Steuereinheit 16 zugeführt werden. Diese berechnet aufgrund der Kontur des Patientenauges 12 eine passende Einstellung der Verstellelemente des Spiegels 36.

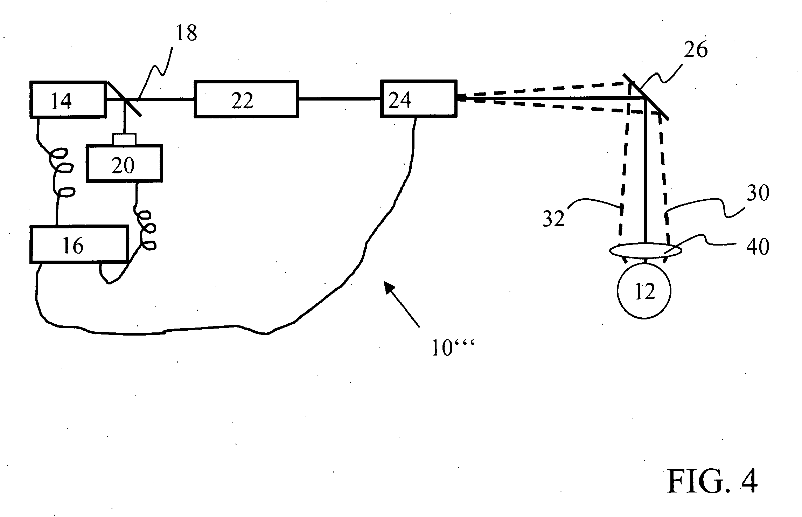

Ein Lasersystem 10''' gemäß einer dritten Ausführungsform der Erfindung ist in Fig. 4 gezeigt. Das Lasersystem 10 aus Fig. 1 wird um eine Linse 40 erweitert, und zwar ist diese Linse so platziert und geformt, dass die seitlich abgelenkten Strahlen 30 und 32 auch hier senkrecht auf der Oberfläche der Hornhaut des Auges 12 auftreffen. Die genaue Einstellung ist sehr empfindlich, so dass man in der Regel die Linse nicht einfach fest in dem Lasersystem 10''' vorsehen kann. Damit die seitlich abgelenkten Strahlen 30 und 32 tatsächlich senkrecht auf der Hornhautoberfläche des Patientenauges 12 auftreffen, muss man die Linse 40 gegebenenfalls verschieben, so dass die Linse 40 verschiebbar ausgebildet sein muss, und sie kann auch so gestaltet sein, dass ihre Form veränderlich ist. Die veränderliche Form der Linse kann insbesondere deren Brennweite verändern, sie kann sogar vom Auftreffpunkt abhängige unterschiedliche Brennweiten definieren.

## Patentansprüche

1. Lasersystem (10', 10") zum Ablatieren von Hornhaut an einem Patientenauge (12) eines Patienten, mit Mittel zur vorbestimmter Platzierung des Patientenauges mit Bezug auf das Lasersystem, mit einem Spiegel, mit einer Zieleinrichtung sowie mit einer Laserquelle (14), die im Betrieb einen Laserstrahl (18) zu der Zieleinrichtung (24) hin aussendet, welche den Laserstrahl nacheinander auf unterschiedliche Stellen der Spiegeloberfläche des Spiegels (34, 36) lenkt,
**dadurch gekennzeichnet, dass**
a) die Spiegeloberfläche derart nicht eben ist oder
b) die Spiegeloberfläche derart veränderlich nicht eben einstellbar ist, dass ein aus dem Lasersystem (10',10") austretender Laserstrahl bei vorbestimmter Platzierung des Patienten relativ zu dem Lasersystem unabhängig von der Einstellung der Zieleinrichtung (24) mit einem Auftreffwinkel auf der Hornhautoberfläche auftrifft, der näher an 90° liegt, als dies bei einem ebenen Spiegel der Fall wäre.

2. Lasersystem (10') nach Anspruch 1,
**dadurch gekennzeichnet,**
**dass** die Spiegeloberfläche sphärozylindrisch ist.

3. Lasersystem (10') nach Anspruch 2,
**dadurch gekennzeichnet,**
**dass** die Spiegeloberfläche sphärisch ist.

4. Lasersystem (10') nach Anspruch 1,
**dadurch gekennzeichnet,**
**dass** die Spiegeloberfläche asphärisch ist.

5. Lasersystem (10') nach Anspruch 4,
**dadurch gekennzeichnet,**
**dass** die Spiegeloberfläche ellipsoidal ist.

6. Lasersystem nach einem der vorhergehenden Ansprüche,
**dadurch gekennzeichnet,**
**dass** dem Spiegel eine Linse nachgeordnet ist.

7. Lasersystem (10"') zum Ablatieren von Hornhaut an einem Patientenauge (12) eines Patienten, mit wie bei Anspruch 1 einer Laserquelle (14), die im Betrieb einen Laserstrahl (18) zu der Zieleinrichtung (24) hin aussendet, welche den Laserstrahl nacheinander auf unterschiedliche Stellen der Spiegeloberfläche des Spiegels (26) lenkt,
**dadurch gekennzeichnet,**
**dass** dem Spiegel (26) eine Linse (40) nachgeordnet ist, die eine veränderliche Form hat, die derart einstellbar ist,
**dass** ein aus dem Lasersystem (10',10") austretender Laserstrahl bei vorbestimmter Platzierung des Patienten relativ zu dem Lasersystem unabhängig von der Einstellung der Zieleinrichtung (24) mit einem so nah wie möglich an 90° liegenden Auftreffwinkel auf der Hornhautoberfläche auftrifft.

8. Lasersystem nach Anspruch 6, das gleichzeitig die Eigenschaft gemäß Anspruch 7 aufweist.

9. Verfahren zum Vorbereiten einer Ablation von Hornhaut an einem Auge (12) eines Patienten mit Hilfe eines Lasersystems (10") nach Anspruch **1, Alternative b),** mit den Schritten:
- Vermessen der Kontur der Hornhautoberfläche des Auges (12),
- Einstellen der Spiegeloberfläche derart, dass ein aus dem Lasersystem austretender Laserstrahl bei vorbestimmter Platzierung des Patienten relativ zu dem Lasersystem unabhängig von der Einstellung der Zieleinrichtung (24) mit einem so nah wie möglich an 90° liegenden Auftreffwinkel auf der Hornhautoberfläche auftreffen wird.

10. Verfahren zum Vorbereiten einer Ablation von Hornhaut an einem Auge (12) eines Patienten mit Hilfe eines Lasersystems (10"') nach Anspruch 7, mit den Schritten:
- Vermessen der Kontur der Hornhautoberfläche des Auges (12),
- Einstellen der Form der Linse (40) derart, dass ein aus dem Lasersystem (10"') austretender Laserstrahl bei vorbestimmter Platzierung des Patienten relativ zu dem Lasersystem (10"') unabhängig von der Einstellung der Zieleinrichtung (24) mit einem so nah wie möglich an 90° liegenden Auftreffwinkel auf der Hornhautoberfläche auftreffen wird.

## Claims

1. Laser system (10', 10") for ablating cornea on a patient's eye (12) of a patient, with means for predetermined placement of the patient's eye with respect to the laser system, with a mirror, with a targeting device as well as with a laser source (14) emitting a laser beam (18) towards the targeting device (24) in operation, which consecutively directs the laser beam to different locations of the mirror surface of the mirror (34, 36),
**characterized in that**
a) the mirror surface is non-planar such that or
b) the mirror surface is variably non-planar adjustable such that with predetermined placement of the patient relative to the laser system a laser beam exiting the laser system (10', 10") is incident on the cornea surface with an angle of incidence closer to 90° than it would be the case with a planar mirror independent of the adjustment of the targeting device (24).

2. Laser system (10') according to claim 1,
**characterized in that**
the mirror surface is spherical-cylindrical.

3. Laser system (10') according to claim 2,
**characterized in that**
the mirror surface is spherical.

4. Laser system (10') according to claim 1,
**characterized in that**
the mirror surface is aspherical.

5. Laser system (10') according to claim 4,
**characterized in that**
the mirror surface is ellipsoidal.

6. Laser system according to anyone of the preceding claims,
**characterized in that**
a lens is disposed downstream of the mirror.

7. Laser system (10"') for ablating cornea on a patient's eye (12) of a patient, with means for predetermined placement of the patient's eye with respect to the laser system, with a mirror, with a targeting device as well as with a laser source (14) emitting a laser beam (18) towards the targeting device (24) in operation, which consecutively directs the laser beam to different locations of the mirror surface of the mirror (26),
**characterized in that**
a lens (40) is disposed downstream of the mirror (26), which has a variable shape, which is adjustable such that
with predetermined placement of the patient relative to the laser system, a laser beam exiting the laser system (10', 10") is incident on the cornea surface with an angle of incidence as close to 90° as possible independent of the adjustment of the targeting device (24).

8. Laser system according to claim 6, which has the characteristic according to claim 7 at the same time.

9. Method for preparing ablation of cornea on an eye (12) of a patient with the aid of a laser system (10") according to claim 1, alternative b), comprising the steps of:
- measuring the contour of the cornea surface of the eye (12),
- adjusting the mirror surface such that with predetermined placement of the patient relative to the laser system, a laser beam exiting the laser system will be incident on the cornea surface with an angle of incidence as close to 90° as possible independent of the adjustment of the targeting device (24).

10. Method for preparing ablation of cornea on an eye (12) of a patient with the aid of a laser system (10"') according to claim 7 comprising the steps of:
- measuring the contour of the cornea surface of the eye (12),
- adjusting the shape of the lens (40) such that with predetermined placement of the patient relative to the laser system (10"'), a laser beam exiting the laser system (10"') will be incident on the cornea surface with an angle of incidence as close to 90° as possible independent of the adjustment of the targeting device (24).

## Revendications

1. Système laser (10', 10"), destiné à l'ablation de la cornée sur l'oeil (12) d'un patient, avec un moyen, destiné au placement prédéfini de l'oeil du patient par rapport au système laser, avec un miroir, avec un dispositif de visée, ainsi qu'avec une source laser (14), qui émet, en fonctionnement, un rayon laser (18) vers le dispositif de visée (24), lequel dirige le rayon laser tour à tour sur différents points de la surface du miroir (34, 36),
**caractérisé en ce que**
a) la surface du miroir n'est pas plane ou que
b) la surface du miroir, variable, n'est pas réglable de manière plane,
de telle sorte qu'un rayon laser, qui émerge du système laser (10', 10"), en cas de placement prédéfini du patient par rapport au système laser, percute la surface de la cornée, indépendamment du réglage du système de visée (24), avec un angle d'incidence qui est plus proche de 90° que cela ne serait le cas avec un miroir plane.

2. Système laser (10') selon la revendication 1,
**caractérisé en ce**
**que** la surface du miroir est sphérocylindrique.

3. Système laser (10') selon la revendication 2,
**caractérisé en ce**
**que** la surface du miroir est sphérique.

4. Système laser (10') selon la revendication 1,
**caractérisé en ce**
**que** la surface du miroir est asphérique.

5. Système laser (10') selon la revendication 4,
**caractérisé en ce**
**que** la surface du miroir est ellipsoïdale.

6. Système laser selon l'une des revendications précédentes,
**caractérisé en ce**
**qu'**une lentille est placée après le miroir.

7. Système laser (10"'), destiné à l'ablation de la cornée sur l'oeil (12) d'un patient, avec un moyen, destiné au placement prédéfini de l'oeil du patient par rapport au système laser, avec un miroir, avec un dispositif de visée, ainsi qu'avec une source laser (14), qui émet, en fonctionnement, un rayon laser (18) vers le dispositif de visée (24), lequel dirige le rayon laser tour à tour sur différents points de la surface du miroir (26),
**caractérisé en ce**
**qu'**une lentille (40) est placée après le miroir (26), laquelle a une forme variable, qui est réglable,
de telle sorte qu'un rayon laser, qui émerge du système laser (10', 10"), en cas de placement prédéfini du patient par rapport au système laser, percute la surface de la cornée,indépendamment du réglage du système de visée (24), avec un angle d'incidence, qui est le plus proche possible de 90°.

8. Système laser selon la revendication 6, qui présente en même temps la propriété conformément à la revendication 7.

9. Procédé de préparation de l'ablation de la cornée de l'oeil (12) d'un patient, à l'aide d'un système laser (10") selon la revendication 1, variante b), avec les étapes consistant à :
- mesurer le contour de la surface de la cornée de l'oeil (12),
- régler la surface du miroir, de telle sorte qu'un rayon laser, qui émerge du système laser, en cas de placement prédéfini du patient par rapport au système laser, percutera la surface de la cornée, indépendamment du réglage du dispositif de visée (24), avec un angle d'incidence le plus proche possible de 90°.

10. Procédé de préparation de l'ablation de la cornée de l'oeil (12) d'un patient, à l'aide d'un système laser (10"') selon la revendication 7, avec les étapes consistant à :
- mesurer le contour de la surface de la cornée de l'oeil (12),
- régler la forme de la lentille (40), de telle sorte qu'un rayon laser, qui émerge du système laser (10"'), en cas de placement prédéfini du patient par rapport au système laser (10"'), percutera la surface de la cornée, indépendamment du réglage du dispositif de visée (24), avec un angle d'incidence le plus proche possible de 90°.
